# EUROPEAN PATENT APPLICATION

(11) **EP 1 080 719 A2**
(43) Date of publication of application: **07.03.2001**
(21) Application number: 00118980.2
(22) Date of filing: 01.09.2000
(51) Int. Cl.: A61K 9/70, A61K 47/24

(54) **Methods for potentiation of efficacy of topical actives by mono-acyl-(lyso)-glycerophosholipids**

(30) Priority: 03.09.1999 US 152305 P; 01.09.2000 US 654421 P
(71) Applicant: Active Organics Inc., Lewisville, Texas 75057 (US)
(72) Inventor: Bishop, Michael, Dallas, Texas 75220 (US); Gillis, Glen, Denton, Texas 76208 (US); Norton, Scott J., Argyle, Texas 76226 (US)
(74) Representative: Wagner, Karl H., Dipl.-Ing.

(57) **Abstract**

Mono-acyl-(lyso)-glycerophospholipids (also known as lysophosphatidyl derivatives; e.g., lysophosphatidyl choline, a lysolecithin; lysophosphatidyl serine, a lysocephalin), are used either alone or in combination with certain other permeation-enhancing compounds, to enhance the penetration of active ingredients into the vertebrate epidermis and thus enhance and/or potentiate the efficacy of topically-applied active components. Other permeation-enhancing compounds which may be included in formulations with mono-acyl-(lyso)-glycerophospholipids include, but are not limited to, salicylates, esters of choline, acyl carnitines, diethoxyglycol, and detergents such as sodium dodecyl sulfate, lecithins and other phosphatidyl compounds, and other surface-active lipid derivatives, such as ceramides and cerebrosides.

## Description

### FIELD OF THE INVENTION

The invention relates to an improvement of current methods used to potentiate the efficacy of topically applied active components and more particularly, the invention is directed to a method for enhancing the efficacy of cosmetic and therapeutic active components by enhancing the permeation of such components across the epidermal barrier.

### Background of the Invention

The mammalian epidermal barrier, also known as the horny layer of the epidermis, is known to consist of dead keratinocytes cemented together by both lipopolysaccharides and proteinaceous linkage components. This barrier protects the organism from the external environment as well as from dehydration. Although this tissue normally serves as an efficient barrier and is apparently well designed for its role, the outer layer of the skin (the horny layer) incurs both 1) non-traumatic damage over time due to natural aging processes (intrinsic damage) and 2) traumatic damage due to exposure to harmful environmental and/or self induced conditions (extrinsic damage). Damage of the latter type results in what is called "aging skin", which can adversely affect the appearance of the individual as well as reduce the effectiveness of the epidermis in its barrier functions.

Aging of the skin is thought to be caused by the prolonged exposure of the skin to 1) intrinsic damage (e.g., normal aging processes), and 2) extrinsic damage such as environmental exposure, e.g., pollution, UV radiation, and subject-induced damage, e.g., smoking and alcohol. A common method of combating these deleterious effects is the topical use of formulations containing active components intended to prevent, protect against, or repair such damage. Examples of such active components include, but are not limited to, antioxidants, anti-inflammatory agents, moisturizers, exfoliating agents, de-pigmenting agents, and texture or color enhancing agents.

The effectiveness of many topically applied active components is limited by their ability to permeate through the epidermal barrier. Many of these active components, particularly the more hydrophilic components, tend to implant or remain upon the outer surface of the skin. Normal processes such as washing, perspiration, and epidermal sloughing tend to remove such stationary components from the skin before they can penetrate the epidermal barrier and perform their intended functions.

Because of the need to deliver active components into the skin, and the desire to deliver them to the skin topically, many attempts have been made to enhance the permeability of active components through the epidermal barrier. Successful attempts to enhance epidermal permeability include the use of "additives" such as salicylic acid, detergents (e.g., sodium lauryl sulfate), and diethoxyglycol components through the epidermal barrier by increasing the fluidity of the normally "rigid" outer layer of the skin, the horny layer.

Although the success of such additives with respect to enhanced epidermal permeation (or penetration) of active components into the skin is well known, they do have shortcomings. Each of the aforementioned components commonly used to enhance epidermal penetration are known to cause irritation to the user when used in topical formulations. It is proposed that irritation, due to the use of effective levels of these permeation enhancers, likely results from the inability of the organism to regulate, modify, or remove these components from the epidermis. Thus, such permeation enhancers remain within the epidermis for extended periods of time and deteriorate the epidermal barrier, resulting in the loss of protection from the external environment and the loss of moisture from the epidermis. The need for a method to enhance the permeation of cosmetic and/or therapeutic active components through the epidermal barrier such that controlled permeation can occur without irritation is obvious to those skilled in the art.

United States Patent 4,164,573 describes a composition for a rectally administered hypoglycemic agent for treatment of diabetes in mammals. Among the components of the composition is a list of physiological surface active agents; one of the many listed is lysolecithin, a mono-acyl-(lyso)-glycerophospholipid. The use of this component is limited, however, to rectal administration of actives.

United States Patent 5,221,696 describes the use of monoacyl phosphoglycerides, both naturally-occurring and synthetic, for the sole use of ocular delivery of ophthalmic drugs, with the purpose being an enhancement of corneal penetration.

United States Patent 5,736,161 describes a pharmaceutical preparation for improving the bioavailability of drugs via oral delivery (mucus membranes). A method is described whereby selected drugs are incorporated into microparticles (millispheres, microspheres, and nanoshperes). A large list of "promoters of absorption" are also components of the pharmaceutical preparation, one of which is lysolecithins. The use of lysolecithins (mono-acyl-[lyso]-glycerophospholipids) in these preparations is limited solely to oral administration.

United States Patent 5,863,554 describes an enhanced drug delivery system for administering an active drug to the nasal mucosal membranes of a vertebrate. The delivery system is composed of bioadhesive microspheres formed of materials selected from the group consisting of proteins, polysaccharides, hyaluronic acid esters, and synthetic polymers, wherein each of the bioadhesive microspheres contains a physiologically effective amount of an active drug and a permeation-enhancing material. The permeation-enhancing material is a biological surfactant selected from the group consisting of bile salts, fatty acids, fatty acid salts, acylglycerols, tyloxapols, acylcarnitines, phospholipids, lysophosphatides, fusidates, and mixtures thereof. Although this patent invokes use of lysophosphatides (in conjunction with specified types of microspheres) as one of a group of surfactants for use in enhancing drug permeation via nasal administration, it does not describe or claim the use of lysophosphatides (mono-acyl-[lyso]-glycerophospholipids) for the enhancement of permeation of active ingredients into the epidermis of vertebrate skin for cosmetic and/or therapeutic delivery purposes.

United States Patent 5,932,562 a method to reduce cholesterol absorption by incorporating selected plant sterols (phytosterols), known to reduce cholesterol absorption, into formulations which may contain either lecithins, lysolecithins, or both. The compositions may be dosed in capsule or tablet form, or by adding either in liquid or dry powder form to foods and beverages. Again, the use of lysolecithins in this patent is solely for oral administration.

United States Patent 6,017,545 relates to a delivery system for the administration of large-molecule pharmaceuticals through oral and nasal mucosal membranes. A large list of absorption enhancing compounds is given, one compound of which is lysolecithin. The patent does not relate to the use of lysolecithin, a mono-acyl-(lyso)-glycerophospholipid, for the purpose of potentiating the efficacy of cosmetic and/or therapeutic active components by enhancing the permeation of such components across the epidermal barrier.

Mono-acyl-(lyso)-glycerophospholipids have been known for a number of years to affect the structure and permeability of synthetic biomembranes. One early study showed that small levels of mono-acyl-(lyso)-glycerophospholipids (e.g., lysolecithins), incorporated into lecithin bilayer vesicles, enhance the permeability of metal ions across the vesicular membrane (Lee and Chan, 1977). It was found in that study that ion permeability was lysolecithin concentration-dependent and was very greatly enhanced at temperatures below the lipid phase transition temperature (Tc). Above the Tc the permeation, while enhanced, was less so than at the lower temperatures. From these data the authors suggested that at temperatures above the Tc lysolecithin forms ion channels within the lipid bilayer involving four lysolecithin molecules per channel. At temperatures below the Tc, it was proposed that long-lived lysolecithin clusters are formed in the membrane bilayer. These clusters, which are structurally perturbed or disordered, serve as channels for rapid ion permeation.

More recently, *in vivo* studies have demonstrated that topical application (to hairless rats) of radioactively labeled mono-acyl-(lyso)-glycerophospholipids (specifically, lysophosphatidyl-cholines) results in a significant penetration of these compounds into the epidermis where they are ultimately metabolized (either enzymatically degraded, or converted to typical membrane phospholipids by enzymatic acylation with a fatty acid). Topical application of mono-acyl-(lyso)-glycerophospholipid caused no damage to skin structure as determined by microscopic examination (Uchida, *et al.,* 1991). The authors suggested that lyso glycerophospholipids, such as lysolecithins, could be useful agents for dermatological use since they have antiviral and bactericidal activity. No suggestion was made, however, of a possible use of these "lyso" compounds to improve the efficacy of, or delivery of, topically applied active components.

Citation or identification of any reference in the background of this application shall not be construed as an admission that such reference is available as prior art to the present invention.

### SUMMARY OF THE INVENTION

The invention involves the novel use of mono-acyl-(lyso)-glycerophospholipids (also known as lysophosphatidyl derivatives; e.g., lysophosphatidyl choline, a lysolecithin; lysophosphatidyl serine, a lysocephalin), either alone or in combination with certain other permeation-enhancing compounds, to enhance the penetration of active ingredients into the vertebrate epidermis and thus enhance and/or potentiate the efficacy of topically-applied active components. Other permeation-enhancing compounds which may be included in formulations with mono-acyl-(lyso)-glycerophospholipids include, but are not limited to, salicylates, esters of choline, acyl carnitines, diethoxyglycol, and detergents such as sodium dodecyl sulfate, lecithins and other phosphatidyl compounds, and other surface-active lipid derivatives, such as ceramides and cerebrosides.

Such permeation-enhancing systems involving mono-acyl-(lyso)-glycerophospholipids may be used in formulations before the active compounds are applied, may be mixed in formulations with the active components and applied at the same time, or may be used in formulations subsequent to the application of the active components. The order of application is determined in part by the type of active compounds being applied, the characteristics of the response desired, and the type of formulation and/or carrier involved.

### Brief Description of the Drawings

FIG. 1 lists reagents used in compositions of the invention;
FIG. 2 shows the composition of a tropical gel used in compositions of the invention;
FIG. 3 shows the composition of a tropical cream used in compositions of the invention;
Fig. 4a shows the composition of a lipstick base according to the invention;
Fig. 4b shows the lipstick base used in conjunction with other elements of a final lipstick composition;
Fig. 5 shows a hydorgel formulation use in a non-occlusive transdermal patch;
FIG. 6 shows the composition of a lip balm;
Table 1A shows the effects of mono-acyl-(lyso)-glycerophospholipids and duration of trans-epidermal water loss;
Table 1B shows the effects of mono-acyl-(lyso)-glycerophospholipids on average increase in trans-epidermal water loss and on irritation scores;
Table 2 shows the efects of various substances on cell renewal and desquamation; and
Table 3 shows enhancement effects of mono-acyl-(lyso)-glycerophospholipids on cell renewal rates.

### DETAILED DESCRIPTION OF THE INVENTION

The invention is directed to the use of mono-acyl-(lyso)-glycerophospholipids, alone, or in combination with other permeation-enhancing agents, to increase the fluidity of the epidermal barrier in a controlled and regulated manner such that active components can more readily and more efficiently penetrate the epidermal barrier without the irritation often associated with the use of permeation enhancement agents in current use.

The term "mono-acyl-(lyso)-glycerophospholipids" generally describes a class of glycerol phospholipids and includes both 1-acyl-2-lyso-glycerophospholipids and 1-lyso-2-acyl-glycerophospholipids. The term "1-acyl-2-lyso-glycerophospholipids" generally describes a class of glycerol phospholipids having the general formula R₁OCH₂₋CHO(R₂) - CH₂OR₃, and pharmaceutically and/or cosmetically acceptable salts thereof wherein R₁ is a hydrocarbyl fatty acid acyl group having 4-26 carbon atoms or a hydrocarbyl heteroatom fatty acid acyl group having 3-25 carbon atoms; R ₂ is a hydrogen atom; R₃ is a naturally occurring polar head group characteristic of a glycerophospholipid isolated from an endogenous source.

The term "1-lyso-2-acyl-glycerophospholipids" generally describes a class of glycerol phospholipids having the general formula of R₁OCH₂₋CHO(R₂)-CH₂OR₃, and pharmaceutically and/or cosmetically acceptable salts thereof wherein R₁ is a hydrogen; R₂ is a hydrocarbyl fatty acid acyl group having 4-26 carbon atoms or a hydrocarbyl heteroatom fatty acid acyl group having 3-25 carbon atoms; R ₃ is a naturally occurring polar head group characteristic of a glycerophospholipid isolated from an endogenous source.

Some specific examples of mono-acyl-(lyso)-glycerophopholipid compounds (utilizing more commonly employed nomenclature) used in conjunction with the invention are: lysophosphatidyl compounds such as lysophosphatidyl-cholines, lysophosphatidyl-serines, lysophosphatidyl-glycerols, lysophosphatidyl-ethanolamines, lysophosphatidyl-inositols, lysophosphatidic acid, etc. This invention is not, however, limited to these examples.

In the development of the invention, families of compounds that enhance, or potentiate, the permeation of active components across the vertebrate epidermal barrier, and thereby enhance the effectiveness of cosmetic and/or therapeutic components in topical formulations were investigated. In the course of these investigations it was found that mono-acyl-(lyso)-glycerophospholipids, when present in topical formulations which may also contain other polar lipids (e.g., phospholipids such as diacylglycerophospholipids; sphingolipids such as cerebrosides), are very effective in promoting and/or enhancing these desired effects.

The use of mono-acyl-(lyso)-glycerophospholipids in formulations as an adjunct to other topical therapies is an improvement over existing technologies because of the following discoveries in our investigations:

The enhancement of permeation of topically applied active components can be made to be quite substantial. The degree of enhancement of permeation of actives may be controlled by the concentration of mono-acyl-(lyso)-glycerophospholipids in formulations that are applied to the skin of the subject; thus, the extent of barrier function perturbation can be limited to a non-irritating level.

The time frame over which the permeation of topical active compounds is enhanced may be controlled by the concentration of mono-acyl-(lyso)-glycerophospholipids applied in formulations to the subject's skin; thus, the extent of the loss of barrier function can be controlled and limited to a non-irritating level.

The mono-acyl-(lyso)-glycerophospholipids used to enhance permeation are either converted to safe by-products and eliminated from the skin tissues layers by natural body processes, or converted into diacyl-glycerophospholipids and incorporated into cell membranes as normal membrane components by natural body processes, thus eliminating accumulation of unmodified residues and the prospect for long-term irritation.

Because the mono-acyl-(lyso)-glycerophospholipids enhance (potentiate) the permeation and effectiveness of topically applied active components, the efficiency of such active components is improved. Thus, the concentration of the active components may be reduced while maintaining efficacy levels. This reduction in the necessary concentrations of active components can provide benefits such as (a) reducing the formulation's overall cost when expensive active components are involved; (b) reducing consumption of rare active components; (c) reducing intensive extraction processes, such as solvent intensive processes, that may be associated with producing active components; (d) reducing packaging constraints; (e) reducing the amount of preservatives required, or eliminating the need for them entirely; and (f) reducing problems related to formulating with greater concentrations of active ingredients, such as maintaining uniform dispersions, managing interactions among active species, and maintaining the appearance, smell, and texture qualities of the end products.

It is pointed out that the cosmetic compositions of the present invention are those which enhance the permeation of active components of the compositions, when administered to the skin. Thus, such cosmetic compositions may improve texture or appearance of the skin, or enhance epidermal exfoliation and/or epidermal cell renewal, or effect other desired responses to the skin (e.g., color, elasticity, moisture, etc.), without necessarily rendering a benefit or an effect of treating or preventing an abnormal biological condition, disease, or disorder. Such improvements or responses are related to the specific active components (compounds or mixtures thereof, extracts or mixtures thereof) in the cosmetic compositions and to their augmented activity when in formulations which promote enhanced permeation through the epidermal barrier. Examples of the cosmetic benefits to the skin that may be associated with the present invention include, but are not limited to, improvements of the texture or appearance of the skin by facilitating active ingredient distribution within the skin. Such enhancement of active ingredient distribution within the skin can provide a natural-looking and/or natural-feeling coating in the skin so as to enhance the beauty and/or smoothness of the skin from its pretreated state. This can also include the providing of a temporary moisturizing effect to the epidermis. Abnormal biological conditions or diseases that may receive temporary cosmetic benefit from the present invention include, but are not limited to dry skin, severe dry skin, dandruff, pruritus, age spots, lentigines, melasmas, wrinkles (both coarse and fine, caused by intrinsic as well as extrinsic damage), warts, blemished skin, hyperpigmented skin, hyperkeratotic skin, inflammatory dermatoses, age-related skin changes and skin in need of cleansers, as well as the effects of skin atrophy, and psoriasis.

It is further pointed out that the therapeutic compositions of the present invention are those which enhance the permeation of therapeutic active components of the compositions into the epidermal and subepidermal levels when administered to the skin. Thus, such therapeutic compositions may render a benefit or an effect of treating or preventing an abnormal condition, disease, or disorder, or the reduction in severity or disappearance of the symptoms or cause of the abnormal condition, disease, or disorder. The reduction in severity or disappearance of the abnormal condition, disease, or disorder may be either in the short- or long-term. Such benefits or effects are related to the specific active components (compounds or mixtures thereof, extracts or mixtures thereof) in the therapeutic compositions and to their augmented activity when in formulations which promote their enhanced permeation through the epidermal barrier. Examples of abnormal biological conditions, diseases, or disorders to be treated by administering therapeutic compositions of the present invention include, but are not limited to, dry skin, severe dry skin, dandrull acne, keratoses, eczema, skin flakiness, pruritus, age spots, lentigines, melasmas, wrinkles (both coarse and fine, caused by intrinsic as well as extrinsic damage), warts, blemished skin, hyperpigmented skin, hyperkeratotic skin, inflammatory dermatoses, age-related skin changes and skin in need of cleanser, as well as the effects of skin atrophy and psoriasis.

A major utility of the present invention is that it encompasses methods for effective cosmetic and/or therapeutic treatment of the skin for the purpose of temporarily improving (cosmetic), or for the purpose of treating/preventing (therapeutic), abnormal conditions, diseases, or disorders of the types (but not limited to those types) that are specified above. The methods comprise the topical administration (and also modes of topical administration) of the cosmetic or therapeutic formulations of the invention. These formulations contain appropriately selected types and amounts of active components and also appropriate amounts of mono-acyl-(lyso)-glycerophospholipids, and are applied to an area of a subject's skin, having the condition, disease, or disorder. The net positive result is an enhancement of desired effect(s) by specific active components at lower concentrations than would otherwise be required. The invention thus also provides additional utilities of (1) lower costs for expensive active components, (2) decreased irritation/inflammation than might be encountered at high concentrations of active components, and (3) greater flexibility in product formulation because of the lower concentrations of the active components.

In another specific embodiment, the present invention provides methods for the enhancement of epidermal exfoliation and/or epidermal cell renewal. The methods comprise a topical administration, to an area of the subject's skin, of a formulation containing a specified amount of an exfoliant (e.g., an alpha hydroxy acid or salicylic acid) in the presence of an appropriate concentration of permeation enhancer, mono-acyl-(lyso)-glycerophospholipids. The concentrations of exfoliant required for the desired exfoliation rate are thereby decreased, resulting in a greatly decreased irritation and/or inflammation of the skin.

In still yet another embodiment, the invention provides methods for enhancing the effects of skin lighteners. The methods are comprised by administering to an area of a subject's skin an effective amount of a formulation which contains an appropriate amount of a skin lightener (e.g, dihydroquinone or kojic acid) in the presence of an appropriate concentration of permeation enhancer, mono-acyl-(lyso)-glycerophospholipids. The concentration of skin lightener normally required for the desired effect is decreased by the presence of the permeation enhancer, decreasing possible irritation/inflammation of the skin.

In still yet another embodiment, the invention provides methods for enhancing cell renewal and desquamation, while concomitantly causing a dramatic decrease in the size of exfoliated squames. The methods are comprised by administering to an area of a subject's skin an effective amount of a composition containing an appropriate amount of an exfoliating agent (e.g., alpha-hydroxy acids, such as L-lactic acid; salicylic acid, etc.) in the presence of an appropriate concentration of permeation enhancer, mono-acyl-(lyso)-glycerophospholipids. An "appropriate" concentration of the permeation enhancer is that which gives the desired enhanced cell renewal rate, and concomitant decrease in size of exfoliated squames, in the presence of each specific exfoliating agent.

In still yet another embodiment, the invention encompasses methods for the preparation of various delivery formulations (e.g., sprays, lotions, gels, creams, salves) of cosmetic and/or therapeutic compositions which contain active components and permeation enhancer, mono-acyl-(lyso)-glycerophospholipids, that are applied topically to a subject's skin to effect the desired response(s).

In still yet another embodiment, the invention encompasses various delivery modes for the application of its formulations to the skin (including, but not limited to: spray application, roller application, direct rub-on application, and application to the skin by use of skin patches [epidermal patches]), to provide the most effective delivery methodologies for formulations of the invention to specific areas of the skin or to specific regions of the body, and that are commensurate with the cosmetic or therapeutic intention of the application.

The compositions and methods of the present invention demonstrate high levels of therapeutic activity, and/or of cosmetic effects, against skin conditions or disorders, heretofore not achieved by the same concentrations of the active components by themselves.

### COMPOSITIONS

The compositions of the present invention provide for treating or preventing abnormal skin conditions, diseases or disorders, and/or for improving the texture or appearance of the skin (e.g., by enhancing epidermal exfoliation and/or epidermal cell renewal or by effecting other desired responses to the skin, such as color, elasticity, moisture, etc.). The compositions comprise one or more active components, described below, and mono-acyl-(lyso)-glycerophospholipids, which enhance the permeation of the cosmetic and/or therapeutic active component(s) across the epidermal barrier of the skin. The mechanism by which the mono-acyl-(lyso)-glycerophospholipids accomplish this desired effect is as follows.

Upon topical application, the mono-acyl-(lyso)-glycerophospholipids are incorporated into the horny layer due to their lipid characteristics and consequently, due to their chemical characteristics, increase the fluidity within the horny layer of the epidermis.

The increased fluidity of the horny layer facilitates the enhancement of permeation and thus the potentiation of the cosmetic and/or therapeutic active components. This permeation enhancement occurs whether these active components are applied before, simultaneously with, or after the formulation containing the mono-acyl-(lyso)-glycerophospholipids is applied.

Over time, the mono-acyl-(lyso)-glycerophospholipids are incorporated into the bilayer membrane of cells and then undergo a process referred to as "lipid recycling", in which the lyso-lipid content of an organism is re-acylated by cellular mechansims as described elsewhere (D. E. Vance and J. E. Vance, ed., 1985).

Upon removal and re-acylation of the mono-acyl-(lyso)-glycerophospholipids from the horny layer of the epidermis, the integrity of the barrier function of the epidermis returns to normal.

The extent of enhancement, or potentiation, of the effects of the active component(s) are regulated by the concentration of the permeation enhancer, mono-acyl-(lyso)-glycerophospholipids, in the formulations of the invention, and achieve the desired results with little or no irritation, inflammation, or other undesired effects, to the skin.

For purposes of this invention, "active components" in cosmetic and/or therapeutic formulations may include, for example, but not by way of limitation, those that improve or eradiate age spots, keratoses, and wrinkles; analgesics; anesthetics; antiacne agents; antibacterials; antiyeast agents; antifungal agents; antiviral agents; antidandruff agents; antidermatitis agents; antipruritic agents; antimetics; antiinflammatory agents; antihyperkeratolytic agents; antidryskin agents; antiperspirants, antipsoriatic agents; antiseborrheic agents; hair conditioners and hair treatment agents; antiaging and antiwrinkle agents; sunscreen agents; skin lightening agents; depigmenting agents; vitamins, corticosteroids, tanning agents; hormones; peptides; retinoids; clotrimazole; hetoconazole; miconazole; griseofulvin; hydroxyzine; diphenhydramine; pramoxine; lidocaine; procaine; mepivacaine; monobenzone; erythromycin; tetracycline; clindamycin; meclocyline; hydroquinone; minocycline; naproxen; ibuprofen; theophylline; nicotine; minoxidil; capsacian; cromylyn; albuterol; retinoic acid; 13-cis-retinoic acid; hydrocortisone; hydrocortisone 21-acetate; hydrocortisone 17-valerate; hydrocortisone 17-butyrate; betamethasone valerate; betamethasone dipropionate; triamcinolone acetonide; fluocinonide; clobetasol propionate; benzoyl peroxide; crotamiton; propranolol; promethazine; vitamin A palmitate; vitamin E acetate.

Further, for purposes of this invention, cosmetic active components are those which are unable to penetrate beyond the epidermis of the skin, whether or not a permeation enhancer is present in the composition which is applied to the skin surface. Therapeutic active components are those which may (but not necessarily) penetrate through the epidermis and into the subepidermal levels of the skin, particularly when the permeation enhancer is present in the composition applied to the skin surface.

"Active components" such as the above examples, and which also apply to this invention, may be contained within extracts of natural materials (e.g., botanical extracts; animal tissue extracts; fungal and microbial extracts). Further, in clarification for the purposes of this invention, the criteria as to whether or not a compound, mixture of compounds, extract or mixture of extracts which may contain a variety of compounds, should be called an "active component" are:
1) the compound, mixture of compounds, or extract(s) must exhibit or potentially exhibit beneficial effect(s) when applied, in an appropriate formulation and in an effective concentration, to the skin. Such applications may be for the treatment or prevention of abnormal skin conditions, diseases or disorders, for the improvement of the texture or appearance of the skin (e.g., by enhancing epidermal exfoliation and/or epidermal cell renewal or by effecting other desired responses such as skin color, elasticity, moisture), or for other desired and beneficial effects to the recipient's biological system that may be attained by therapeutically active components when delivered across the epidermis (subepidermal delivery); and
2) the desired cosmetic and/or therapeutic response elicited by the active component is made possible or is enhanced (potentiated) by the permeation enhancer, mono-acyl-(lyso)-glycerophospholipids, when the latter is present in appropriate concentrations in formulations containing the "active component". Use of the terms, "enhanced" and "potentiated", for the purposes of the present invention, mean that a) the concentration of active component in cosmetic (epidermal) compositions or therapeutic (epidermal and/or subepidermal) compositions that is required for the desired response(s) to the skin surface (epidermal) or to other organ systems (epidermal and/or subepidermal) is decreased when in the presence of the permeation enhancer; and/or b) the desired effects given by an apparently optimal concentration of active component, in compositions which do not contain permeation enhancer, are measurably increased (made more effective, more desirable, or more visible) when in the presence of the permeation enhancer.

The examples of active components listed above are given for illustration purposes only; active components that may be utilized in this invention are not limited to these examples.

Another way of expressing the meaning of the term "active component" is: any molecule or group of molecules, extract or group of extracts that, when present in formulations of the present invention, is made more efficient in its desired cosmetic and/or therapeutic action by the permeation enhancement activities of the permeation enhancer, mono-acyl-(lyso)-glycerophospholipids. The concentrations of active components in formulations of the present invention may vary from a small fraction of one percent to multiple per cents by weight of the final composition, depending on the amount typically required for desired cosmetic and/or therapeutic responses and further, on the degree of enhancement (potentiation) of their efficiency by the presence of permeation enhancer, mono-acyl-(lyso)-glycerophospholipids.

The epidermal permeation enhancer in the compositions of the present invention may be a single molecular type (e.g., lysolecithins), or a mixture of molecular types (e.g., lysolecithins, lysophosphatidyl serines, lysophosphatidyl ethanolamines, lysophosphatidyl glycerols, lysophosphatidyl inositols), all of which may classified as mono-acyl-(lyso)-glycero-phospholipids. The mono-acyl-(lyso)-glycerophospholipids in the compositions of the present invention may be derived by synthetic methods, or may be derived from any source known to those of skill in the art, including for example, natural sources such as plants (found naturally or derived by processes involving genetic engineering processes/techniques), animals, fungi, bacteria and other microbes (whether they are found in nature or are derived from processes involving genetic engineering processes/techniques). The mono-acyl-(lyso)-glycerophospholipids utilized in the present invention may also be in either pure form or in the presence of other molecular species, including, but not limited to, polar lipids (such as lecithins, and other phosphatidyl derivatives) and/or nonpolar lipids.

The epidermal permeation enhancer, mono-acyl-(lyso)-glycerophospholipids, can be present in compositions of the invention in an amount of about 0.001% to about 99.99% by weight of the final composition, preferably in an amount of about 0.01% to about 10% by weight of the final composition, and most preferably in an amount of about 0.1% to about 5% by weight of the final composition.

The compositions of the present invention, intended for topical application, may contain carrier, excipient, or vehicle ingredients such as, for example, water, acetone, ethanol, ethylene glycol, propylene glycol, butane-1,3-diol, isopropyl myristate, isopropyl palmitate, mineral oil, and mixtures thereof to form lotions, tinctures, creams, emulsions, gels, or ointments which are non-toxic and cosmetically, therapeutically and dermatologically acceptable. Additionally, moisturizers or humectants can be added to the present compositions if desired. Examples of such additional ingredients useful for cosmetic compositions can be found in CTFA International Cosmetic Ingredient Dictionary, Fourth Edition, J.M. Nikitakis, Ed., The Cosmetic, Toiletry, and Fragrance Association, Washington, D.C., 1991.

The present invention provides methods for enhancing epidermal exfoliation and/or for enhancing epidermal cell renewal. Such methods comprise topically administering to an area on a subject's skin an effective amount of a composition of the present invention, which comprises an appropriate concentration of epidermal permeation enhancer, mono-acyl-(lyso)-glyderophospholipids, and an appropriate concentration of active component, the exfoliation/cell renewal effects of which are enhanced (potentiated) by the presence of the permeation enhancer. The composition is also administered in a manner (or mode) and with a frequency such that the effectiveness of epidermal exfoliation and/or epidermal cell renewal are further enhanced.

The present invention also provides methods for improving the texture and/or appearance of skin. Such methods comprise administering to an area of a subject's skin an effective amount of a composition of the present invention, which comprises an appropriate amount of epidermal permeation enhancer, mono-acyl-(lyso)-glycerophospholipids, and an active component, the skin texture/appearance improvement effects of which are enhanced (potentiated) by the presence of the permeation enhancer. The composition is also administered in a manner (or mode) and with a frequency such that the desired cosmetic effects are further enhanced.

The present invention also provides methods for treating or preventing an abnormal skin condition, disease, or disorder. Such methods comprise administering to an area of a subject's skin an effective amount of a composition of the present invention, which comprises an appropriate amount of epidermal permeation enhancer, mono-acyl-(lyso)-glycerophospholipids, and an active component, the treatment/preventative effects of which are enhanced (potentiated) by the presence of the permeation enhancer. The composition is also administered in a manner (or mode) and with a frequency such that the desired treatment/preventative effects are further enhanced. Such condition, disease, or disorder includes, but is not limited to, dry skin, severe dry skin, dandruff, acne, keratoses, eczema, skin flakiness, pruritus, age spots, lentigines, melasmas, wrinkles (both coarse and fine, caused by intrinsic as well as extrinsic damage), warts, blemished skin, hyperpigmented skin, hyperkeratotic skin, inflammatory dermatoses, age-related skin changes and skin in need of cleanser, as well as the effects of skin atrophy and psoriasis.

A preferred method of administering an effective amount of a composition of the present invention for any of the methods described above is via topical application. The amount of active component and of epidermal permeation enhancer, mono-acyl-(lyso)-glycerophospholipids, in the final composition, and the frequency of topical application to the skin can vary widely, depending upon factors such as the particular skin disorder, the severity of the skin disorder, the location and/or type of the skin involved, the subjects skin sensitivity, and the degree of treatment desired. It is well with the purview of the skilled artisan to regulated dosages according to the subject's need(s). It is suggested as an example that topical application range from about once per week to about 4 or 5 times daily, preferably from about 3 times a week to about 3 times daily, most preferably about once or twice per day. The concentrations of active components in formulations of the present invention may vary from a small fraction of one percent to multiple per cents by weight of the final composition, depending on the amount typically required for desired cosmetic and/or therapeutic responses and further, on the degree of enhancement (potentiation) of their efficiency when in the presence of permeation enhancer, mono-acyl-(lyso)-glycerophospholipids. The epidermal permeation enhancer, mono-acyl-(lyso)-glycerophospholipids, can be present in compositions of the invention in an amount of about 0.001% to about 99.99% by weight of the final composition, preferably in an amount of about 0.01% to about 10% by weight of the final composition, and most preferably in an amount of about 0.1% to about 5% by weight of the final composition.

### Examples of the Invention

In order to more fully illustrate the nature of the invention and the matter of practicing the same, the following examples are provided, which are not to be construed as limiting the remainder of the disclosure or the scope of the invention in any way whatsoever.

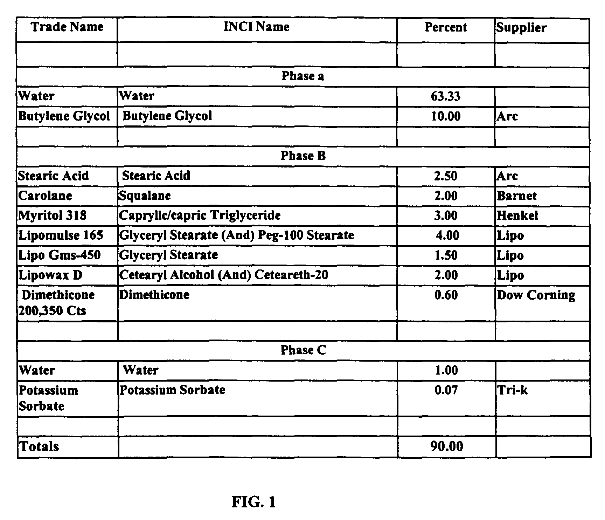

FIG. 1 shows a listing of reagents used in the compositions described above. Reagents were mixed in a manner well known to those skilled in the art of formulation. To the resulting base cream, the additions as listed in the Tables 1A and 1B, Table 2, and Table 3 were admixed. Samples were then titrated with an appropriate concentrated solution of hydrochloric acid (6M) or sodium hydroxide (30%w/v) to the appropriate pH (pH=3.5). Samples were then brought to a 100% volume by the addition of water.

This permeation enhancement system may be formulated into pharmaceutical, cosmetic, beauty aid, or hair care compositions intended for topical use. These compositions include, but are not limited to, topical medications, analgesics, anti-fungals, creams, lotions, gels, salves, balms, foundations, deodorants, colorants, hand lotions, body lotions, moisturizers, lipsticks, lip balms, lip glosses, eye shadows, eye liner, mascaras, sun screens, sun lotions, shampoos, hair conditioners, and hair styling products. This permeation enhancement system or carriers containing it may be formulated into solid or roll-on configurations similar to those used for ordinary deodorant products. They also may be infused into devices such as sponges; swabs, pads, application packs, bandages, or films, or used in mechanical sprayers, atomizers, or misters. The formulation or device containing this permeation enhancement system is applied or used similarly to the way any similar topical product is applied or used.

The general approach to formulating with the permeation enhancement system is similar to those used to make topical formulations. The type of configuration used and the ingredients included with this permeation enhancement system will depend in part on how the formulation is to be applied and whether the permeation enhancement is to be initiated before the active permeate is introduced, at the same time the active permeate is introduced, or after the active permeate is introduced. The following are two general examples of formulations where the active ingredient (retinol palmitate) is formulated with this permeation enhancement system.

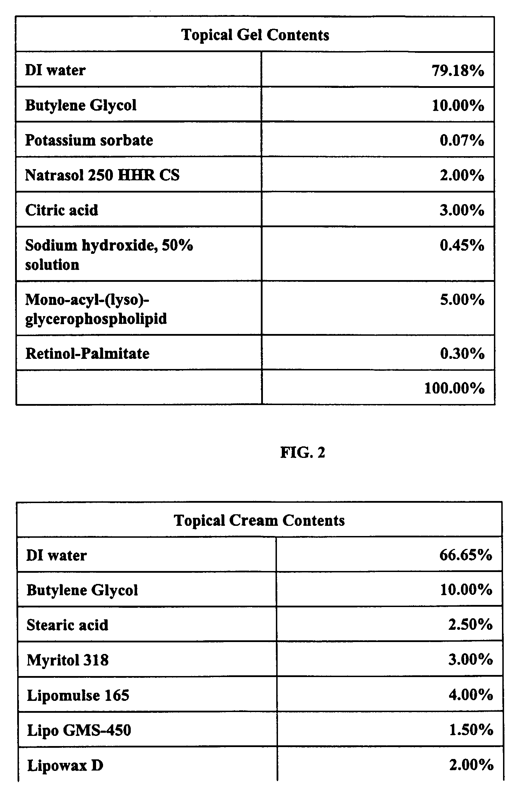

FIG. 2 shows the composition of a topical gel. The ingredients shown in FIG. 2 are mixed in the indicated percentages to formulate the topical gel. The mono-acyl-(lyso)-glycerophospholipid is added to the mixture in amount to be approxixmately 5% of the topical gel mixture.

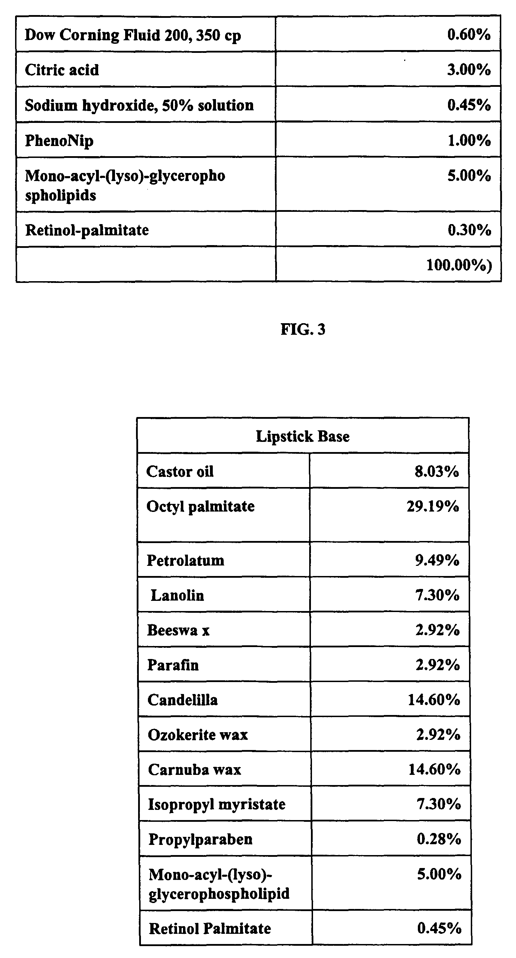

FIG. 3 shows the composition of a topical cream. The ingredients shown in FIG. 3 are mixed in the indicated percentages to formulate the topical cream. The mono-acyl-(lyso)-glycerophospholipid is added to the mixture in amount to be approximately 5% of the topical cream mixture.

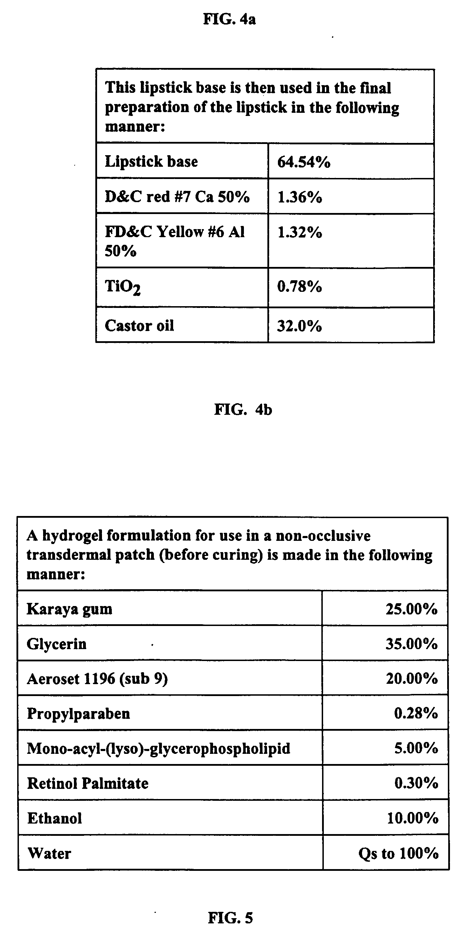

FIG. 4a shows the composition of a lipstick base. In this composition, the lipstick base is made using mono-acyl-(lyso)-glycerophospholipid in an amount to be approximately 5% of the of the lipstick base. The lipstick base is made mixing the components while heating to 80° C, and continuing until the mixture is clear.

A lipstick is then made using the lipstick base composition and the components shown in FIG. 4b. The lipstick base is used in the final preparation of the lipstick, mixing the components shown in FIG. 4a. The components are mixed while heating to 80°C, mixing until uniform the composition is uniform.

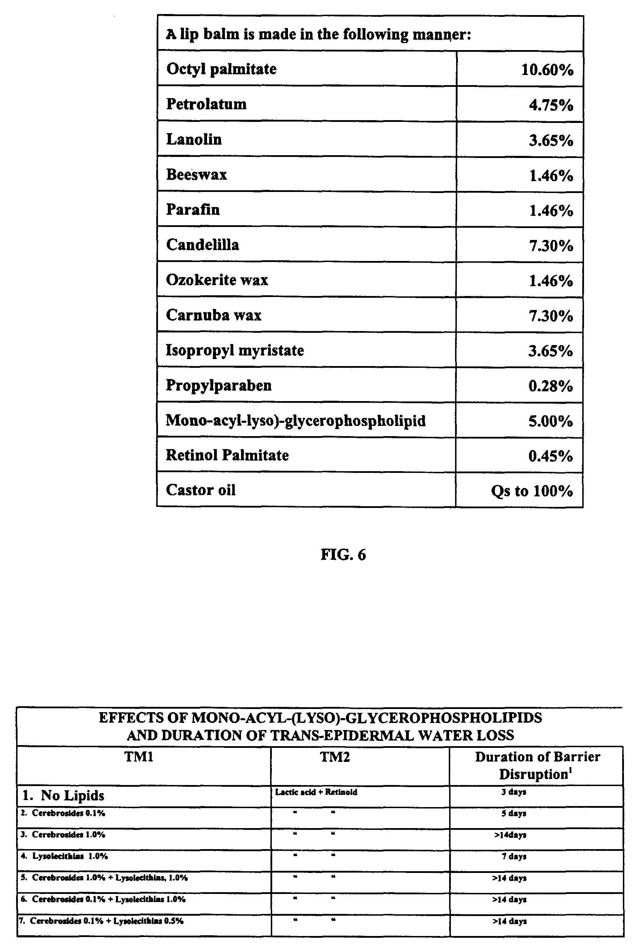

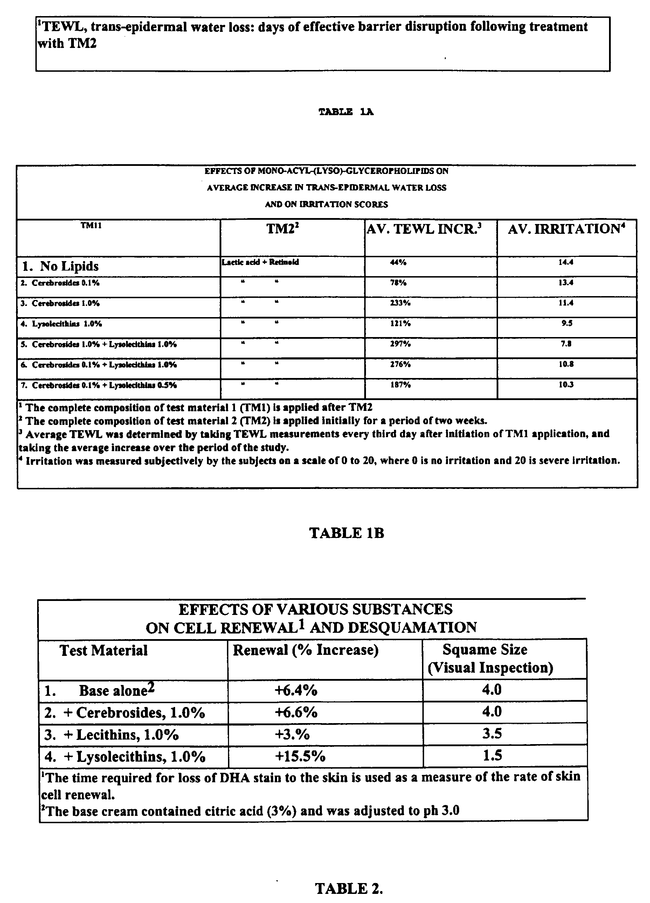

Another composition is shown in FIG. 5. This composition is a hydrogel formulation for use in a non-occlusive transdermal patch (before curing).

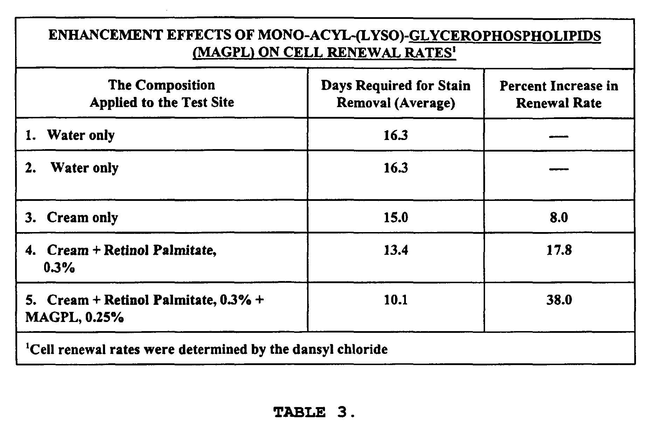

FIG. 6 shows a composition use in a formulation for a lip balm.

The formulations of the invention show an improved efficacy of active components that promote skin cell exfoliation/cell renewal when in the presence of mono-acyl-(lyso)-glycerophospholipids.

One of the compositions of the present invention which contains alpha-hydroxy acid (L-lactic acid) and retinol palmitate was tested in the presence and absence of cerebrosides and lysolecithins. High concentrations of alpha-hydroxyacids and of retinoids perturb, or disrupt, the epidermal water barrier, and thereby, it is proposed, promote epidermal exfoliation and/or cell renewal. Certain lipids, such as ceramides, increase the duration of the water barrier perturbation, but only at concentrations that are cost-prohibitive. The tests summarized in Tables 1A and 1B were designed to determine if mono-acyl-(lyso)-glycerophospholipids (in this study lysolecithins) would potentiate the effects of cerebrosides when the latter are present in lower, non cost-prohibitive concentrations.

Two test materials (TM1 and TM2) were employed in the study summarized in Tables 1A and 1B. TM2, which contained L-lactic acid, 8%, and retinol palmitate, 0.5%, in a composition at pH 3.0, was applied initially, on a daily basis, to the lower leg of 6 subjects for a period of two weeks. This treatment gave an approximate 3-fold increase in epidermal barrier disruption over controls at the end of the two week period. Then TM1, which contained in composition cerbrosides alone, lysolecithins alone, cerebrosides and lysolecithins in combination, or no lipid at all, at pH 7.0, was applied for an additional two week period. The extent of epidermal barrier disruption during this two week period was determined every three days by measuring trans-epidermal water loss (TEWL). Of particular interest in the study was the length of time barrier disruption was extended by a continued two week treatment with the components of TM1 following termination of treatment with TM2.

The subjects selected for clinical studies of the present invention were between the ages of 20 and 55 years and were required to refrain from using any products on their volar forearm and/or on their lower leg, except those supplied in conjunction with the test procedure for 5 day before and during the test period. None of the subjects: 1) was pregnant or lactating or intended to become pregnant within three months following the test; 2) had one or more known communicable diseases; 3) was involved in any other clinical study at the time of the test; 4) suffered from psoriasis, dermatitis, and/or eczema; 5) was taking medication considered likely to interfere with the test results, including retinoids, corticosteroids, or antibiotics during the six months prior to the test; and/or 7) had used tobacco products, had sensitive skin, had a skin type deemed unsuitable for the test, or ad other characteristics considered likely to make them unsuitable for the test.

Trans-epidermal water loss, TEWL, was measured using the Evaporometer EP1 with a 2105 probe. Prior to testing, subjects were allowed to equilibrate for 30 minutes in an isolation room set at defined relative humidity (typically between 30-40%) and temperature (68-72C). Any cleansing of the skin was completed at least two hours prior to the test, according to the protocol. Tested sites were exposed to the air with no covering of clothing. After calibration of the probe according to manufacturer's instructions, the probe was applied lightly to the skin surface and measurements were recorded after approximately 20-30 seconds, or until the signal stabilized. The millivolt output signal was either recorded by the technician or captured via an RS32 interface and stored on a diskette in a data storage program. Three readings were made at each test site, and if variation with any reading was more than 40%, the measurement was discarded and repeated. The three readings were averaged and recorded as the data point.

It has been reported (United States Patent 5,720,963) that prolonged disruption of the stratum corneum barrier leads to antiaging effects on the skin. Further, it was shown that while a combination of alpha-hydroxy acids and retinoids can efficiently disrupt this barrier, the disruption effect typically lasts for only 5 days or so, due apparently to an augmentation in skin lipid production. Cerebrosides extend the duration of barrier disruption, possibly by inhibiting new lipid production. The enhancement of the cerebroside effect by mono-acyl-(lyso)-glycerophospholipids (lysolecithins) is quite dramatic in the study summarized in Tables 1A and 1 B. This enhancement offers an attractive alternative to the use of very expensive cerebrosides alone to extend barrier disruption for the consequent increased antiaging effects. Thus, as shown in the above study, the effects of lower (and therefore much less expensive) concentrations of the active component, cerebrosides, can be greatly enhanced (potentiated) by supplementation of cosmetic/therapeutic compositions with the relatively inexpensive permeation enhancer, mono-acyl-(lyso)-glycerophospholipids. Furthermore, the irritation decrease found when cerebrosides alone are added to the lactic acid-retinoid composition is even more pronounced when lysolecithins (alone, or supplemental to the cerebrosides) are present (right column, Table 1B).

In another clinical study of the present invention, the effects of mono-acyl-(lyso)-phospholipids (in this study, lysolecithin) on exfoliation rate and on the relative size of exfoliated squames were determined. This is shown in Table 2. The study involved a comparison with other lipids, as well as with the well known exfoliant, lactic acid. Exfoliation enhancement was determined by use of dihydroxyacetone (DHA). Using DHA to stain the upper layers of the epidermis, and by following the time required for loss of the stain (the faster the loss rate, the greater the rate of exfoliation/cell renewal), the ability of lysolecithins to enhance exfoliation of the skin was determined and compared with that of other well known lipids. Details of the DHA procedure are virtually identical with those of the dansyl chloride method (described below).

The analysis of the relative sizes of squames involves the removal of cell clumps from the treated skin area by washing and rubbing with water or surfactant (0.5% sodium dodecylsulfate) in a retaining device. The squames removed are visually inspected for size under a microscope, and the sizes rated as 0 for single cells through 5 for the largest clumps. The squames sizes shown in Table 2 represent average values resulting from several individual readings. It is generally recognized that the smaller the exfoliated squames, the better the resultant visual cosmetic effect.

Mono-acyl-(lyso)-glycerophospholipids 1) potentiate the effects of cerebrosides on prolonging the time-length of epidermal water barrier disruption, 2) potentiate the effects of cerebrosides in enhancing the magnitude of barrier disruption (as measured by trans-epidermal water loss values), and 3) decrease skin irritation by alpha-hydroxyacids and retinoids more efficiently than do cerebrosides (See Tables 1A and 1B). Further, as is shown in Table 2, mono-acyl-(lyso)-glycerophospholipids when added to the base cream significantly stimulate cell renewal, and equally important, they decrease the size of exfoliated squames which is associated with enhanced cosmetic effects. The base cream alone, which contains 3% citric acid, a mild exfoliating agent, gives a low but measureable increase in percent cell renewal. A greatly enhanced percent cell renewal response, and greatly decreased exfoliated squames size, result when mono-acyl-(lyso)-glycero- phospholipids are added to the base cream; the addition of cerebrosides alone or lecithins alone to the base cream give no significant response change.

In yet another clinical study of the present invention, the effects of mono-acyl-(lyso)-glycerophospholipids on the enhancement of efficiency of epidermal cell renewal rates by the active component, retinylpalmitate, were determined. The mono-acyl-(lyso)-glycerophospholipids were derived from a soy extract by a proprietary enzymatic process. The concentration of mono-acyl-(lyso)-glycerophospholipids in the extracts was determined by high performance liquid chromatography. The evaluation of relative exfoliation rates/cell renewal rates was made using a dansyl chloride procedure. This method involves staining the stratum corneum with fluorescent dansyl chloride by applying semi-occlusive patches of 5% dansyl chloride milled into petrolatum for 24 hours. After the 24 hour period, assurance that the stain was completely taken up by the stratum corneum layers was made by viewing under a quartz mineral lamp (short and long ultraviolet wavelength). The 10 subjects in this study were selected according to the aforementioned subject selection procedure (Section 5.3). Each subject was thusly treated with 3 patches of dansyl chloride on each volar forearm and, after the 24 hour period, the sites were examined to insure uniform uptake of the fluorescent stain; the subjects were then instructed to apply the test material to each specific site (a total of 5 sites were actually used) according to a defined daily procedure. Two sites were used as control sites, where water alone was applied; one application site was for the application of the formulation cream alone; and the other two sites were utilized for applications of two formulations creams, one containing active component and the other containing active component plus permeation enhancer. The subjects were uninformed as to the composition of the three formulation creams. The subjects were requested to return on day 7 following the initial applications, and then to return thereafter as required until all the stains had disappeared. The cell renewal rates were then determined on the basis of the number of days required for complete stain disappearance. The results of this clinical study are summarized in Table 3.

The findings of this study, summarized in Table 3, clearly demonstrate that the cell renewal rate given by the active component, retinylpalmitate (composition 4), is dramatically enhanced (over two fold), when 0.25% permeation enhancer, mono-acyl-(lyso)-glycerophospholipids, is present along with the retinylpalmitate (composition 5).

These example clinical studies support the claims of the present invention, specifically that mono-acyl-(lyso)-glycerophospholipids potentiate the efficacy of topically applied active components. Further, methods have been provided for enhancing the efficacy of cosmetic and/or therapeutic active components via enhancement of permeation of such active components across the epidermal water barrier. Finally, mono-acyl-(lyso)-glycerophospholipids, whether alone or in concert with other permeation enhancers (e.g., cerebrosides), provide methodologies for increasing the fluidity of the epidermal barrier in a controlled and regulated manner, such that active components can more readily and more efficiently penetrate the epidermal barrier without the irritation often associated with the use of permeation enhancement agents in current use.

### SUMMARY OF THE INVENTION

1. A composition for increasing the permeability of a mammalian epidermal body surface and a cellular membrane by the use of at least one active component in combination with mono-acyl-(lyso)-phosphoglycerides as a permeation enhancer.
2. The composition where the active component consists of an effective concentration of at least one therapeutically effective drug that includes, but is not limited to, those drugs that act at the epidermal layers of the mammal and those drugs that act at the sub-epidermal layers of the mammal.
3. A composition wherein the therapeutically effective drug is a steroidal hormone and includes, but is not limited to, at least one of the following; progesterone and derivatives thereof, estrogen and derivatives thereof, and testosterone and derivatives thereof.
4. A composition wherein the therapeutically effective drug is a peptide that will act as a hormone and includes, but is not limited to, at least one of the following; melatonin, AGHA, IGF, interleukin, interferon, MSH, a-TGF, b-TGF, TNF and MCH.
5. A composition wherein the therapeutically effective drug is a lipid that will act as a hormone and includes, but is not limited to, at least one of the following; PDGF, retinoiic acid, leukotrienes, and prostaglandins.
6. A composition wherein the therapeutically effective drug is a nucleic acid based polymer and includes, but is not limited to, at least one of the following; DNA based polymers and RNA based polymers.
7. A composition wherein the therapeutically effective drug is a analgesic and includes, but is not limited to, at least one of the following; acetylsalicylic acid, ibuprofen, and acetaminophen, capsaicin, menthol, and methylsalicylate.
8. A composition wherein the therapeutically effective drug is a treatment for arthritis and includes, but is not limited to, at least one of the following, menthol, methylsalicylate, and capsaicin.
9. A composition wherein the therapeutically effective drug is a treatment to induce vasodilatation and includes, but is not limited to, at least one of the following; sildenafil, arginine, and compounds that are known to effect the levels and action of nitric oxide in a mammal.
10. A composition wherein the therapeutically effective drug is a treatment for addiction and includes but is not limited to, nicotine.
11. A composition wherein the therapeutically effective drug is a treatment for depression and other mental diseases and disabilities and includes, but is not limited to, at least one of the following; naltrexone, fenfluramine, valium, fluoxetine, methylphenidate, MAO inhibitors, and seratonin re-uptake inhibitors.
12. A composition wherein the therapeutically effective drug is a treatment for cancer.
13. A composition wherein the therapeutically effective drug is a treatment for heart disease and includes, but is not limited to, at least one of the following; nitroglycerin, drugs known to decrease blood cholesterol levels.
14. A composition wherein the therapeutically effective drug is a treatment for bacterial infection and includes, but is not limited to, at least one of the following; penicillin and derivatives thereof, erythromycin, tetracycline, sulfur based antibacterials, and berberine and other alkaloid based antibacterials.
15. A composition wherein the therapeutically effective drug is a treatment for viral infection and includes, but is not limited to, at least one of the following; AZT and interferon.
16. A composition wherein the therapeutically effective drug is a treatment for parasitic infestation and includes but is not limited to, ivermectin.
17. A composition wherein the therapeutically effective drug is a treatment for fungal infestation.
18. A composition wherein the therapeutically effective drug acts as an antioxidant and includes, but is not limited to, at least one of the following; epigallocatechin gallate, epicatechin gallate, propyl gallate, proanthrocyanadines, anthrocyanidines, ferrulic acid, NDGA, and caffeic acid.
19. A composition wherein the therapeutically effective drug acts as a delivery agent for oxygen and includes but is not limited to, perfluorodecalin.
20. A composition wherein the therapeutically effective drug acts as a treatment to diminish hyperpigmentation and includes, but is not limited to, at least one of the following; hydroquinone, arbutin, kojic acid, ascorbic acid, ascorbyl-phosphate salts, and derivatives thereof.
21. A composition wherein the therapeutically effective drug acts as an exfoliant and includes, but is not limited to, at least one of the following; salacylic acid, lactic acid, glycolic acid, citric acid, malic acid, and proteolytic enzymes,
22. A composition wherein the therapeutically effective drug acts as a superficial skin peel and includes, but is not limited to, at least one of the following; salacylic acid, lactic acid, glycolic acid, citric acid, malic acid, and proteolytic enzymes.
23. A composition wherein the therapeutically effective drug acts as a medium depth skin peel and includes, but is not limited to, trichloroacetic acid.
24. A composition wherein the therapeutically effective drug acts as a deep peel and includes, but is not limited to, phenol.
25. A composition wherein the therapeutically effective drug acts as an astringent.
26. A composition wherein the therapeutically effective drug acts as a humectant.
27. A composition wherein the therapeutically effective drug acts as an isoflavone and includes, but is not limited to, at least one of the following; genistin, genistein, daidzin, daidzein, glycitin, and glycetein.
28. A composition wherein the therapeutically effective drug acts as an terpenoid and includes, but is not limited to, at least one of the following; ursolic acid, oleanolic acid, betulinic acid, glyrrzyretinnic acid, and derivatives thereof.
29. A composition wherein the therapeutically effective drug acts as an vitamin and includes, but is not limited to, at least one of the following; vitamin A and derivatives and precursors thereof, vitamin C and derivatives and precursors thereof, vitamin D and derivatives and precursors thereof, vitamin E and derivatives and precursors thereof, and vitamin K and derivatives and precursors thereof.
30. A composition wherein the permeation enhancer is present in concentrations ranging from 0.01% to 99.99%.
31. A composition wherein the permeation enhancer is combined with a permeation-enhancing amount of at least one other known permeation enhancer.
32. A composition wherein the permeation enhancer and the therapeutically effective drug are dispersed in an acceptable pharmaceutical carrier.
33. A composition wherein the permeation enhancer and the therapeutically effective drug are incorporated into a medical device, such as a transdermal patch, designed to enhance the penetration of a therapeutically effective drug.
34. A composition where the active component consists of at least one cosmetically beneficial component that, when applied topically to the epidermis induces a cosmetically beneficial therapeutic effect.
35. A composition wherein the cosmetically beneficial component improves the structure of the skin of a mammal.
36. A composition wherein the cosmetically beneficial component, when applied topically, results in the appearance of younger looking skin.
37. A composition wherein the cosmetically beneficial component diminishes the appearance and degree of clinically apparent wrinkles.
38. A composition wherein the cosmetically beneficial component is one that repairs the existing intrinsic damage to the skin.
39. A composition wherein the cosmetically beneficial component is one that inhibits the appearance of intrinsic damage to the skin.
40. A composition wherein the cosmetically beneficial component is one that prevents the appearance of intrinsic damage to the skin.
41. A composition wherein the cosmetically beneficial component is one that repairs the existing extrinsic damage to the skin.
42. A composition wherein the cosmetically beneficial component is one that inhibits the appearance of extrinsic damage to the skin.
43. A composition wherein the cosmetically beneficial component is one that prevents the appearance of extrinsic damage to the skin.
44. A composition wherein the cosmetically beneficial component is a steroid and includes, but is not limited to, at least one of the following; progesterone and derivatives thereof, estrogen and derivatives thereof, and testosterone and derivatives thereof.
45. A composition wherein the cosmetically beneficial component is a peptide and includes, but is not limited to, at least one of the following; melatonin, AGHA, IGF, interleukin, interferon, MSH, a-TGF, b-TGF, TNF and MCH.
46. A composition wherein the cosmetically beneficial component is a lipid and includes, but is not limited to, at least one of the following; PDGF, retinoiic acid, leukotrienes, and prostaglandins.
47. A composition wherein the cosmetically beneficial component is a nucleic acid based polymer and includes, but is not limited to, at least one of DNA based polymers and RNA based polymers.
48. A composition wherein the cosmetically beneficial component is a analgesic and includes, but is not limited to, at least one of acetylsalicylic acid, ibuprofen, and acetaminophen, capsaician, menthol, and methylsalicylate.
49. A composition wherein the cosmetically beneficial component is a treatment for arthritis and includes, but is not limited to, at least one of menthol, methylsalicylate, and capsaician.
50. A composition wherein the cosmetically beneficial component is a treatment to cause vasodilatation and includes, but is not limited to, at least one of sildenafil, arginine, and compounds that are known to effect the levels and action of nitric oxide in a mammal.
51. A composition wherein the cosmetically beneficial component is a treatment for the prevention of cancer.
52. A composition wherein the cosmetically beneficial component is a treatment for bacterial infection and includes, but is not limited to, at least one of penicillin and derivatives thereof, erythromycin, tetracycline, sulfur based antibacterials, and berberine and other alkaloid based antibacterials.
53. A composition wherein the cosmetically beneficial component is a treatment for viral infection and includes, but is not limited to, at least one of AZT and interferon.
54. A composition wherein the cosmetically beneficial component is a treatment for parasitic infestation and includes, but is not limited to, ivermectin.
55. A composition wherein the cosmetically beneficial component is a treatment for fungal infestation.
56. A composition wherein the cosmetically beneficial component acts as an antioxidant and includes, but is not limited to, at least one of the following; epigallocatechin gallate, epicatechin gallate, propyl gallate, proanthrocyanadines, anthrocyanidines, ferrulic acid, NDGA, and caffeic acid.
57. A composition wherein the cosmetically beneficial component acts as a delivery agent for oxygen and includes but is not limited to, perfluorodecalin.
58. A composition wherein the cosmetically beneficial component acts as a skin lightener and includes, but is not limited to, at least one of the following; hydroquinone, arbutin, kojic acid, ascorbic acid, ascorbyl-phosphate salts, and derivatives thereof.
59. A composition wherein the cosmetically beneficial component acts as a moisturizer and includes, but is not limited to, at least one of the following;
60. A composition wherein the cosmetically beneficial component acts as an exfoliant and includes, but is not limited to, at least one of the following; salacylic acid, lactic acid, glycolic acid, citric acid, malic acid, and proteolytic enzymes.
61. A composition wherein the cosmetically beneficial component acts as a superficial skin peel and includes, but is not limited to salacylic acid, lactic acid, glycolic acid, citric acid, malic acid, and proteolytic enzymes.
62. A composition wherein the cosmetically beneficial component acts as a medium depth skin peel and includes, but is not limited to, trichloroacetic acid.
63. A composition wherein the cosmetically beneficial component acts as a deep peel and includes, but is not limited to, phenol.
64. A composition wherein the cosmetically beneficial component acts as an astringent.
65. A composition wherein the cosmetically beneficial component acts as a humectant.
66. A composition wherein the cosmetically beneficial component is an isoflavone and includes, but is not limited to, at least one of the following; genistin, genistein, daidzin, daidzein, glycitin, and glycetein.
67. A composition wherein the cosmetically beneficial component is an terpenoid and includes, but is not limited to, at least one of the following; ursolic acid, oleanolic acid, betulinic acid, glyrrzyretinnic acid, and derivatives thereof.
68. A composition wherein the cosmetically beneficial component is an vitamin and includes, but is not limited to, at least one of the following; vitamin A and derivatives and precursors thereof, vitamin C and derivatives and precursors thereof, vitamin D and derivatives and precursors thereof, vitamin E and derivatives and precursors thereof, and vitamin K and derivatives and precursors thereof.
69. A composition wherein the permeation enhancer is present in concentrations ranging from 0.01% to 99.99%.
70. A composition wherein the permeation enhancer is combined with a permeation-enhancing amount of at least one other known permeation enhancer.
71. A composition wherein the permeation enhancer and the cosmetically beneficial component are dispersed in an acceptable cosmetic carrier.
72. A composition wherein the permeation enhancer and the cosmetically beneficial component are incorporated into a cosmetic device, such as a transdermal patch, designed to enhance the penetration of a cosmetically beneficial component
73. A composition wherein the mono-acyl-(lyso)-phospholipid includes, but is not limited to, at least one of the following; lysolecithins, lysophosphatidylcholine, lysophosphatidyl serines, lysophosphatidyl ethanolamines, lysophosphatidyl glycerols, and lysophosphatidyl inositols.
74. A composition wherein the mono-acyl-(lyso)-phospholipid includes, but is not limited to, at least one of the following; those derived from synthetic sources, those derived from natural sources, and those derived from bioengineered sources.
75. A composition wherein the mono-acyl-(lyso)-phospholipid includes, but is not limited, to at least one of the following; those derived from plant sources, mammalian sources, reptilian sources, aquatic sources, bacterial sources, fungal sources, viral sources, and yeast.
76. A composition wherein the mono-acyl- (lyso)-phospholipid may be in pure form.
77. A composition wherein the mono-acyl- (lyso)-phospholipid may be in the presence of other molecular species, including, but not limited to, at least one of the following groups; polar lipids (such as lecithins, and other phosphatidyl derivatives), nonpolar lipids, vegetable oils, surfactants, and emulsifiers.
78. A composition wherein the mono-acyl-(lyso)-phospholipid may be in Compositions of the invention containing therapeutically effective drugs in an amount of about 0.001% to about 99.999% by weight of the final composition, preferably in an amount of about 0.01% to about 10.00% by weight of the final composition, and most preferably in an amount of about 0.1% to about 2.0% by weight of the final composition.
79. A composition wherein the mono-acyl-(lyso)-phospholipid may be in compositions of the invention containing cosmetically beneficial components in an amount of about 0.001% to about 99.999% by weight of the final composition, preferably in an amount of about 0.01% to about 10.00% by weight of the final composition, and most preferably in an amount of about 0.1% to about 5.0% by weight of the final composition.
80. A composition wherein the mono-acyl-(lyso)-phospholipid is utilized in the formula to control the rate of permeation of an active compound through the epidermal barrier such that it affects the rate of action of said active compound in a time controlled manner. 81. A composition for improving the general health, appearance and condition of the skin, comprising:
   at least one active ingredient employed for improving the general health, appearance and condition of the skin; and
   an epidermal permeation enhancer composed of mono-acyl-(lyso)-glycerophospholipids, that potentiates the efficiency of the active ingredient(s) used for improving the general health, appearance and condition of the skin.
82. The composition wherein the active ingredient is comprised of one of a single active compound and active compounds derived from at least one of natural, synthetic sources, and extracts derived from plant, animal, fungal, or microbial sources that contain the desired active compound(s).
83. The composition wherein the active ingredient is present in an amount, by weight of the final composition, to effect the desired cosmetic/therapeutic response when its efficiency is potentiated by appropriate amounts of the permeation enhancers, mono-acyl-(lyso)-glycerophospholipids.
84. The composition wherein the permeation enhancer, mono-acyl-(lyso)-glycerophospholipids, is derived from at least one of products of synthetic processes and products derived from natural sources including extracts of plants, animals, fungi and microbes.
85. The composition wherein the mono-acyl-(lyso)-glycerolphospholipids include at least one of a cosmetically and therapeutically acceptable carrier, vehicle and excipient.
86. The composition wherein the at least one cosmetically and therapeutically acceptable carrier, vehicle, and excipient component of the mono-acyl-(lyso)-glycerophospholipids is selected from a group consisting of lotions, tinctures, creams, emulsions, gels, ointment, water, water-workable cream, polyvinyl alcohol, hydroxyethyl cellulose, cellulose, hydrophilic acrylic polymer, emollients, skin moisturizing components, stabilizers, glycerol, surfactants, preservatives, hydrophilic thickening agents use in cosmetic and skin therapeutic formulations and mixtures thereof.
87. The composition wherein the mono-acyl-(lyso)-glycerophospholipids are present in an amount of from 0.001% to 30% by weight of the final composition.
88. The composition wherein the mono-acyl-(lyso)-glycerophospholipids are present in an amount from 0.01% to 5% by weight of the final composition.
89. The composition wherein the mono-acyl-(lyso)-glycerophospholipids are present in an amount of 0.1% to 2% by weight of the final composition.
90. A method for treating and preventing an abnormal skin condition, disease, disorder, and for regulating the effects of skin atrophy and improving the texture or appearance of the skin which comprises topically administering to a subject on an area of skin by one of a direct application and by use of an (epidermal patch), an effective amount of a composition comprising:
   an active ingredient known to have a desired effect for the treatment, prevention or improvement of the foregoing conditions and situations; and
   mono-acyl-(lyso)-glycerophospholipids, that potentiates the efficiency of the active ingredient selected for the desired effect for the treatment, prevention or improvement of the foregoing conditions and situations.
91. The method wherein the active ingredient is comprised of one of a single active compound and mixture of active compounds derived from at least one of a natural and synthetic sources, extracts derived from plant, animal, fungal, or microbial sources that contain the desired active compound(s).
92. The method wherein the active ingredient is present in an amount, by weight of the final composition, sufficient to effect the desired cosmetic and therapeutic response when its efficiency is potentiated by appropriate amounts of the permeation enhancer, mono-acyl-(lyso)-glycerophospholipids.
93. The method wherein the permeation enhancer, mono-acyl-(lyso)-glycerophospholipids, is derived from at least one of products of synthetic processes and products derived from natural sources including extracts of plants, animals, fungi and microbes.
94. The method according to claim 93 wherein the mono-acyl-(lyso)-glycerolphospholipids further comprise at least one of a cosmetically and therapeutically acceptable carrier, vehicle and excipient.
95. The method wherein one of a the cosmetically and therapeutically acceptable carrier, vehicle and excipient component of the mono-acyl-(lyso)-glycerophospholipids is selected from the group consisting of lotions, tinctures, creams, emulsions, gels, ointment, water, water-workable cream, polyvinyl acrylic polymer, emollients, skin moisturizing components, stabilizers, glycerol, surfactants, preservatives, hydrophilic thickening agents use in cosmetic and skin therapeutic formulations and mixtures thereof.
96. The method wherein the mono-acyl-(lyso)-glycerophospholipids are present in an amount from 0.001% to 30% by weight of the final composition.
97. The method wherein the mono-acyl-(lyso)-glycerophospholipids are present in an amount from 0.01% to 5% by weight of the final composition.
98. The method wherein the mono-acyl-(lyso)-glycerophospholipids are present in an amount from 0.1% to 2% by weight of the final composition.
99. The method wherein abnormal skin condition, disease or disorder is selected from the group consisting of dry skin, severe dry skin, dandruff, acne, keratoses, psoriasis, eczema, skin flakiness, pruritus, age spots, lentigines, melasmas, wrinkles, warts, blemished skin, hyperpigmented skin, hyperkeratotoic skin, inflammatory dermatoses, age-related skin changes, and psoriasis.
100. A method for improving skin texture and appearance, and for enhancing the skin cell renewal rate, which comprises topically administering to a subject on an area of skin an effective amount of a composition, comprising:
   the active ingredient retinol palmitate in the amount of 0.3% by weight of the final composition; and
   mono-acyl-(lyso)-glycerophospholipids in the amount of 0.25% by weight of the final composition.
101. A composition for improving the health, appearance and condition of the skin, comprising:
   at least one active ingredient for improving at least one of the health, appearance and condition of the skin; and
   an epidermal permeation enhancer composed of mono-acyl-(lyso)-glycerophospholipids.
102. A method to increase the epidermal permeation, comprising the steps of:
   selecting an active ingredient used in improving the health, appearance and condition of the skin; and
   mixing the active ingredient with mono-acyl-(lyso)-glycerophospholipids.
103. A method of increasing epidermal permeation of an active component on a transdermal patch, comprising the steps of:
   applying amono-acyl-(lyso)-glycerophospholipid to the transdermal patch; and
   applying the transdermal patch to an area of skin through which the active component is to be absorbed.

## Claims

1. A composition for increasing the permeability of a mammalian epidermal body surface and a cellular membrane by the use of at least one active component in combination with mono-acyl-(lyso)-phosphoglycerides as a permeation enhancer.

2. The composition according to claim 1 where the active component consists of an effective concentration of at least one therapeutically effective drug that includes, but is not limited to, those drugs that act at the epidermal layers of the mammal and those drugs that act at the sub-epidermal layers of the mammal.

3. A composition according to claim 2 wherein the therapeutically effective drug is a steroidal hormone and includes, but is not limited to, at least one of the following; progesterone and derivatives thereof, estrogen and derivatives thereof, and testosterone and derivatives thereof.

4. A composition according to claim 2 wherein the therapeutically effective drug is a peptide that will act as a hormone and includes, but is not limited to, at least one of the following; melatonin, AGHA, IGF, interleukin, interferon, MSH, a-TGF, b-TGF, TNF and MCH.

5. A composition according to claim 2 wherein the therapeutically effective drug is a lipid that will act as a hormone and includes, but is not limited to, at least one of the following; PDGF, retinoiic acid, leukotrienes, and prostaglandins.

6. A composition according to claim 2 wherein the therapeutically effective drug is a nucleic acid based polymer and includes, but is not limited to, at least one of the following; DNA based polymers and RNA based polymers.

7. A composition for improving the general health, appearance and condition of the skin, comprising:
at least one active ingredient employed for improving the general health, appearance and condition of the skin; and
an epidermal permeation enhancer composed of mono-acyl-(lyso)-glycerophospholipids, that potentiates the efficiency of the active ingredient(s) used for improving the general health, appearance and condition of the skin.

8. A composition for improving the health, appearance and condition of the skin, comprising:
at least one active ingredient for improving at least one of the health, appearance and condition of the skin; and
an epidermal permeation enhancer composed of mono-acyl-(lyso)-glycerophospholipids.

9. A method to increase the epidermal permeation, comprising the steps of:
selecting an active ingredient used in improving the health, appearance arid condition of the skin; and
mixing the active ingredient with mono-acyl-(lyso)-glycerophospholipids.

10. A method of increasing epidermal permeation of an active component on a transdermal patch, comprising the steps of:
applying amono-acyl-(lyso)-glycerophospholipid to the transdermal patch; and
applying the transdermal patch to an area of skin through which the active component is to be absorbed.
